# DEMANDE DE BREVET EUROPEEN

(11) **EP 4 316 269 A1**
(43) Date de publication de la demande: **07.02.2024**
(21) Numéro de dépôt: 23020251.7
(22) Date de dépôt: 25.05.2023
(51) Int. Cl.: A23L 33/00, A23L 33/105, A23L 33/115, A23L 33/15

(54) **ENSEMBLE DE GÉLULES À SOULAGER LE STRESS**

(30) Priorité: 01.08.2022 BE 202200049
(71) Demandeur: Laboratoire Puressentiel S.A., 1050 Bruxelles (BE)
(72) Inventeur: Pacchioni, Isabelle, BE-1050 Bruxelles (BE)
(74) Mandataire: Theunis, Patrick

(57) **Abrégé**

L'invention se rapporte à un ensemble de deux gélules, une première gélule, comprenant des huiles essentielles et végétales liquides, des extraits secs de plantes, des vitamines et minéraux, et une deuxième gélule comprenant des extraits secs de plantes et des minéraux. La première gélule est une gélule à double parois, dans laquelle les huiles essentielles et huiles végétales liquides sont comprises dans un compartiment intérieur, et les extraits secs de plantes, les vitamines et les minéraux sont compris dans un compartiment extérieur, sans contact entre les ingrédients des deux compartiments.

L'ensemble des gélules sert à traiter ou soulager les symptômes liés au stress et à la nervosité, et/ou pour stimuler la relaxation, en particulier la relaxation sommeil.

Ce système permet de réunir en une seule gélule et donc en une seule prise facilitée, des actifs qui n'ont pas de contact entre eux évitant ainsi le risque d'interaction qui pourrait altérer l'efficacité de chacun des actifs compris dans les 2 compartiments de la gélule. Ce système permet également une libération en 2 temps des actifs afin d'obtenir un effet de longue durée en libérant les actifs de façon différée et différente selon qu'ils sont compris dans le 1^{er} compartiment extérieur ou le 2^{ème} compartiment intérieur de la gélule.

## Description

### Domaine technique de l'invention

La présente invention concerne un ensemble de deux gélules, respectivement de deux types de gélules.

Ces gélules peuvent être utilisé pour traiter ou soulager les symptômes liés au stress et à la nervosité.

### Contexte de l'état de la technique

On recherche depuis de nombreuses années dans l'industrie pharmaceutique, des produits majoritairement à base de principes actifs médicamenteux issus de la chimie de synthèse, permettant de traiter ou de soulager les symptômes liés au stress et à la nervosité.

De telles compositions provoquent des effets secondaires indésirables et la balance bénéfices risques n'est pas en leur faveur.

Il est fait référence aux documents suivants :
1) Pujara N D et al « Innercap Technology: An innovative approach for Pharmaceutical and Biopharmaceutical Use", Pharmaceutical Process Development, No. 1, 26 Décembre 2012, XP 093025991, ISSN: 2320-1029;
2) "Delivery system solutions: innovative suppléments to support healthy ageing", 30 janvier 2018, XP093026326;
3) "Liquid-filled hard capsules - ready-to-go formulations", 1 janvier 2020, XP093026296;
4) "Liquid-filled capsules dosage form solutions", 1 janvier 2021, XP093026286 ;
5) « Synactifs som green sommeil 30 gélules », 13 novembre 2020, pages 1-8, XP093025815 ;
6) Auga-Bascou Justine : « Prise en charge du stress à l'officine en période COVID-19 », 23 mars 2022, pages 1-89, XP093025662 ;
7) FR 2 878 163 (R&D Pharma (MC) 26 Mai 2006.

Toutefois, aucun document de l'art antérieur ne présente un ensemble de deux gélules comprenant des suppléments nutritionnels, comme décrit ci-après selon la présente invention.

### Objet de l'invention

La présente invention vise à résoudre ces problèmes. Dans ce but, les inventeurs ont entamé des recherches afin de développer des compositions, en forme de gélules, de prise facile permettant une bonne observance du traitement, pour traiter lesdits problèmes de santé, à base d'ingrédients d'origine purement naturelle.

### Brève description de l'invention

L'invention se rapporte à un ensemble de deux gélules,
- une première gélule, comprenant
   - des huiles essentielles et huiles végétales liquides et
   - des extraits secs de plantes, et
   - des vitamines et minéraux, et
- une deuxième gélule comprenant
   - des extraits secs de plantes et des minéraux,
caractérisé en ce que
la première gélule est une gélule à double parois, dans laquelle
- les huiles essentielles et huiles végétales liquides sont comprises dans un compartiment intérieur,
et
- les extraits secs de plantes, les vitamines et minéraux sont compris dans un compartiment extérieur,
- sans contact entre les ingrédients des deux compartiments.

Selon un mode préféré de l'invention, l'ensemble des deux gélules est caractérisé en ce que les huiles essentielles et huiles végétales liquides sont comprises dans le compartiment intérieur de la première gélule (à double parois) à l'état pur, sans addition d'excipient non actif dans la formule. Ce système permet de réunir en une seule gélule et donc en une seule prise facilitée, des actifs qui n'ont pas de contact entre eux évitant ainsi le risque d'interaction qui pourrait altérer l'efficacité de chacun des actifs compris dans les 2 compartiments de la gélule. Ce système permet également une libération en 2 temps des actifs afin d'obtenir un effet de longue durée en libérant les actifs de façon différée et différente selon qu'ils sont compris dans le 1^{er} compartiment extérieur ou le 2^{ème} compartiment intérieur de la gélule.

Selon un mode plus préféré de l'invention, l'ensemble des deux gélules est caractérisé en ce que
- les huiles essentielles liquides comprennent une huile essentielle de mandarine verte et de lavande vraie, et/ou
- les huiles végétales liquides comprennent l'huile de tournesol, et/ou
- les vitamines comprennent au moins la vitamine B6, et/ou
- les minéraux comprennent au moins le magnésium marin, et/ou
- les extraits secs de plantes comprennent un extrait sec de racine de rhodiole.

Selon un mode encore plus préféré de l'invention, l'ensemble des gélules comprend chacun des ingrédients énumérés ci-dessus.

Cet ensemble de deux gélules est d'origine purement naturelle.

Cet ensemble de deux gélules sert à traiter ou à soulager les symptômes liés au stress et à la nervosité.

L'invention, ainsi que ses modes d'application préférés, est décrit dans les revendications dépendantes annexes.
D'autres modes de réalisation préférés et avantages de l'invention sont décrits dans la description suivante.

### Description détaillée de l'invention

La présente invention concerne un ensemble de deux gélules ou de deux 'types' de gélules.
La première gélule ou le premier 'type' de gélule comprend
- des huiles essentielles et huiles végétales liquides et
- des extraits secs de plantes, et
- des vitamines et des minéraux.

La deuxième gélule ou le deuxième 'type' de gélule comprend
- des extraits secs de plantes et des minéraux.
L'ensemble se caractérise vis-à-vis l'état de la technique en ce que
la première gélule est une gélule à double parois, dans laquelle
- les huiles essentielles et huiles végétales liquides sont comprises dans un compartiment intérieur, et
- les extraits secs de plantes, avec les vitamines et les minéraux, sont compris dans un compartiment extérieur,
- sans contact entre les ingrédients des deux compartiments.

Selon un mode plus préféré de l'invention, le magnésium marin compris dans la première gélule, à double parois, est un oxyde de magnésium.

Selon un mode plus préféré de l'invention, dans la deuxième gélule les extraits secs de plantes comprennent un extrait sec d'Eschscholtzia et/ou un extrait sec de racines de Valériane, et/ou les minéraux comprennent un oxyde de magnésium.

Selon un mode plus préféré de l'invention, la première et/ou la deuxième gélule comprend en outre un ou plusieurs des ingrédients suivants :
- un agent de charge, de préférence une cellulose microcristalline ;
- un agent anti-agglomérant, de préférence du stéarate de magnésium ;
- une gélule végétale, de préférence du hypromellose.

Selon un mode plus préféré de l'invention, les gélules comprennent les ingrédients suivants dans les quantités suivantes, quantité indiquée pour une gélule :
pour la première gélule:
   - le magnésium marin entre 100 et 160 mg, dont magnésium entre 50 et 90 mg ;
   - l'huile essentielle de mandarine verte entre 7 et 15 mg ;
   - l'huile essentielle de lavande vraie entre 7 et 15 mg ;
   - l'extrait sec de racine de rhodiole entre 5 et 12 mg ;
   - la vitamine B6 entre 1 et 5 mg ;
pour la deuxième gélule:
   - extrait secs de parties aériennes fleuries d'eschscholtzia entre 5 et 210 mg ;
   - le magnésium marin entre 110 et 170 mg, dont oxyde de magnésium entre 55 et 95 mg ;
   - l'extrait sec de racine de valériane entre 80 et 160 mg.

Selon un mode préféré de l'invention, les deux gélules comprennent chacun les ingrédients cités ci-dessus.

### Utilisation de l'ensemble des deux gélules

L'ensemble des deux gélules selon l'invention sert à traiter ou à soulager les symptômes liés au stress et à la nervosité. L'ensemble sert également à stimuler la relaxation, en particulier la relaxation sommeil.
L'ensemble selon l'invention peut être utilisé à ce fin.

Selon un mode préféré de l'utilisation de l'ensemble des deux gélules, la première gélule est à prendre le matin et la deuxième gélule est à prendre le soir, de préférence après le diner.
De préférence, une cure est recommandée pour soulager, voir traiter les problèmes de santé décrits ci-dessus.
Une telle cure permet de favoriser substantiellement la santé des personnes traitées. Dans une telle cure, l'ensemble des gélules est à prendre pendant une période de 10 à 20 jours, de préférence pendant environ 15 jours.

Les produits disponibles actuellement sur le marché pour lutter contre le stress et la nervosité comprennent une combinaison de huiles essentielles et extraits de plantes, ou une combinaison d'oligoéléments/minéraux et des extraits de plantes, mais pas la combinaison complète avec les trois catégories d'ingrédients actifs de l'ensemble des deux gélules selon la présente invention, notamment les huiles essentielles, les extraits de plantes et les vitamines/minéraux.

### Description détaillée de quelques ingrédients de l'ensemble des gélules selon l'invention :

### Gélule :

Dans le contexte de la présente invention, l'on comprend par « gélule' une capsule à enveloppe dure, désignant une forme galénique d'un médicament ou d'un complément alimentaire, solide, que l'on avale par voie orale. Elle est constituée d'une enveloppe dure, creuse qui contient la ou les substances actives.

La première gélule de l'ensemble des deux gélules selon l'invention est une gélule à double parois.

Selon l'invention, la première gélule est une gélule à double parois, comprenant une gélule intérieure (ou compartiment intérieur) dans une gélule extérieure (ou compartiment extérieur). L'usage d'une telle forme de gélule permet de contrôler le mode de libération des composants de cette gélule. Dans ce cas spécifique les composants ou ingrédients du compartiment extérieur sont libérés et absorbés en premier lieu, les composants et ingrédients compris dans le compartiment intérieur étant libérés dans une deuxième phase.

Ce système permet de réunir en une seule gélule et donc en une seule prise facilitée, des actifs qui n'ont pas de contact entre eux évitant ainsi le risque d'interaction qui pourrait altérer l'efficacité de chacun des actifs compris dans les 2 compartiments de la gélule.

Ce système permet également une libération en 2 temps des actifs afin d'obtenir un effet de longue durée en libérant les actifs de façon différée et différente selon qu'ils sont compris dans le 1^{er} compartiment extérieur ou le 2^{ème} compartiment intérieur de la gélule.

### Huile essentielle :

Aux fins de la présente invention on entend par huile essentielle : une essence volatile extraite de plantes aromatiques ou d'organe de cette plante par la distillation par entraînement à la vapeur d'eau.

### Huile essentielle de Mandarine verte :

L'huile essentielle de Mandarine verte sert à la relaxation et à la détente. Apaisante, elle soulage l'esprit et facilite le sommeil. Le nom botanique est Citrus reticulata Blanco.

Partie de la plante extraite : Péricarpes du fruit (zestes) encore verts. Elle est obtenue par Expression à froid puis centrifugation.

### Huile essentielle de lavande vraie :

Dans le contexte de la présente invention, l'on entend par lavande vraie la `lavandula angustifolia' ou lavande officinale.

### Extrait sec de Rhodiola :

La Rhodiola (Rosea L.) est une plante adaptogène qui permet d'améliorer la résistance de l'organisme contre la nervosité et le de stress.

### Vitamine B6 :

La vitamine B₆ est une vitamine hydrosoluble représenté par trois formes principales : la pyridoxine, le pyridoxal et la pyridoxamine. Dans le présent cas, la pyridoxine est la forme préférée.

### Magnésium marin

Le magnésium marin est extrait du sel de mer.

En tant que nutriment/minéral, le magnésium va de pair avec certains autres, par exemple la vitamine B6. Il va aider à la métabolisation de ces vitamines, et en retour, elles vont améliorer l'absorption du magnésium.

### Eschscholtzia :

Eschscholzia est un genre de la famille des Papaveraceae (la famille du pavot somnifère et du coquelicot) qui comprend notamment le pavot de Californie (Eschscholzia californica) cultivé pour ses propriétés somnifères ainsi que dans les jardins pour une utilisation ornementale.

L'eschscholtzia est employé dans le traitement des troubles du sommeil. Cette plante aide à s'endormir et améliore la qualité de nos nuits. Elle s'avère utile en cas de surmenage nerveux, d'angoisse, d'anxiété.

La partie aérienne (tige, fleur, graine) de la plante est utilisée.

### Agents de charge :

Les agents de charge sont des additifs alimentaires ajoutés dans une denrée alimentaire en vue d'accroître son volume sans pour autant augmenter, de façon significative, sa valeur calorifique ou nutritionnelle. Ils servent à charger la préparation alimentaire, c'est-à-dire, à lui donner du volume sans toutefois dominer la saveur des aliments. Ces composés, autres que l'eau et l'air, sont également appelés agents de remplissage ou agents gonflants. Le polydextrose (E 1200), le mannitol (E 965), le maltitol (E 421) et la gomme arabique ou gomme d'acacia (E414) sont des additifs utilisés comme agents de charge.

### Huiles végétales :

Selon un mode préféré de l'invention, l'huile végétale présente dans le compartiment intérieur de la première gélule, est l'huile végétale de tournesol. Il s'agit d'un excipient non actif ; la présence de cette huile aide à la dilution des huiles essentielles.

De préférence, une gélule comprend entre 100 et 200 mg de cette huile végétale.

## Revendications

1. Ensemble de deux gélules,
- une première gélule, comprenant
- des huiles essentielles et huiles végétales liquides, et
- des extraits secs de plantes, et
- des vitamines et minéraux, et
- une deuxième gélule comprenant
- des extraits secs de plantes et des minéraux,
**caractérisé en ce que**
la première gélule est une gélule à double parois, dans laquelle
- les huiles essentielles et huiles végétales liquides sont comprises dans un compartiment intérieur, et
- les extraits secs de plantes, les vitamines et minéraux sont compris dans un compartiment extérieur,
- sans contact entre les ingrédients des deux compartiments.

2. Ensemble selon la revendication 1, **caractérisé en ce que**
les huiles essentielles et huiles végétales liquides sont comprises dans le compartiment intérieur de la première gélule (à double parois) à l'état pur, sans addition d'excipient non actif dans la formule.

3. Ensemble selon la revendication 1 ou 2, **caractérisé en ce que**
- les huiles essentielles liquides comprennent une huile essentielle de mandarine verte et de lavande vraie, et/ou
- les huiles végétales liquides comprennent l'huile de tournesol, et/ou
- les extraits secs de plantes comprennent un extrait sec de racine de rhodiole.

4. Ensemble selon l'une quelconque des revendications précédentes, **caractérisé en ce que** la première gélule (à double parois) comprend en tant que vitamines au moins la vitamine B6, et/ou en tant que minéraux, au moins un oxyde de magnésium.

5. Ensemble selon l'une quelconque des revendications précédentes, **caractérisé en ce que** dans la deuxième gélule les extraits secs de plantes comprennent un extrait sec d'Eschscholtzia et/ou un extrait sec de racines de Valériane, et/ou les minéraux comprennent un oxyde de magnésium.

6. Ensemble selon l'une quelconque des revendications précédentes, **caractérisé en ce que** la première et/ou la deuxième gélule comprend en outre un ou plusieurs des ingrédients suivants :
- un agent de charge, de préférence une cellulose microcristalline ;
- un agent anti-agglomérant, de préférence du stéarate de magnésium ;
- une gélule végétale, de préférence du hypromellose.

7. Ensemble selon l'une quelconque des revendications précédentes, **caractérisé en ce que** les gélules comprennent les ingrédients suivants dans les quantités suivantes, quantité indiquée pour une gélule :
pour la première gélule:
- le magnésium marin entre 100 et 160 mg, dont oxyde magnésium entre 50 et 90 mg ;
- l'huile essentielle de mandarine verte entre 7 et 15 mg ;
- l'huile essentielle de lavande vraie entre 7 et 15 mg ;
- l'extrait sec de racine de rhodiole entre 5 et 12 mg ;
- la vitamine B6 entre 1 et 2 mg ;
pour la deuxième gélule:
- extrait secs de parties aériennes fleuries d'eschscholtzia entre 5 et 210 mg ;
- le magnésium marin entre 110 et 170 mg, dont oxyde de magnésium entre 55 et 95 mg ;
- l'extrait sec de racine de valériane entre 80 et 160 mg.

8. Ensemble selon l'une quelconque des revendications 1 à 7 pour son utilisation pour traiter ou soulager les symptômes liés au stress et à la nervosité, et/ou pour stimuler la relaxation, en particulier la relaxation sommeil.

9. Ensemble selon la revendication 8, **caractérisée en ce que** la première gélule est à prendre le matin et la deuxième gélule est à prendre le soir, de préférence après le diner.

10. Ensemble selon une des revendications 8 ou 9, **caractérisée en ce que** l'ensemble des gélules est à prendre pendant une période de 10 à 20 jours, de préférence pendant environ 15 jours.
